# EUROPEAN PATENT SPECIFICATION

(11) **EP 0 599 950 B1**
(45) Date of publication and mention of the grant of the patent: **14.05.1997**
(21) Application number: 92917959.6
(22) Date of filing: 24.08.1992
(51) Int. Cl.: A61B 19/00, A61F 2/46

(54) **IMPROVED TOOL FOR REMOVAL OF PLASTICS MATERIAL**
WERKZEUG ZUR ENTFERNUNG VON PLASTISCHEM MATERIAL
INSTRUMENT AMELIORE POUR ENLEVER DE LA MATIERE PLASTIQUE

(30) Priority: 24.08.1991 GB 9118307
(43) Date of publication of application: 08.06.1994
(60) Divisional application: 95101332.5
(73) Proprietor: YOUNG, Michael John Radley, Ashburton, South Devon TQ13 7JX (GB); BRADNOCK, Brian R. D. P., Edinburgh EH9 2DT (GB)
(72) Inventor: YOUNG, Michael John Radley, Ashburton, South Devon TQ13 7JX (GB); BRADNOCK, Brian R. D. P., Edinburgh EH9 2DT (GB)
(74) Representative: Gregory, Timothy Mark
(86) International application number: GB9201553
(87) International publication number: WO9303676

(56) References cited:
- WO-A-90/10423
- DE-C- 2 741 107
- GB-A- 2 229 660

## Description

The present invention relates to an improved tool for use in removal of plastics material. A tool of this general type is disclosed in our British Patent No. GB2229660 but further improvements have been discovered. The tool is particularly but not exclusively, useful in removing plastics cement from such bores in bones as may be used in hip, or other joint, replacements (hereinafter referred to, for convenience, as hip joint replacements).

In a hip joint replacement operation, a metal implant is provided with a long projection which is inserted into a hole drilled in the medulla of the femur and is held firmly in place by means of a plastics cement. On average, such replacements can be expected to last five to ten years. However, due to repetitive shearing forces during daily use, either the bone/cement interface or the cement/metal interface may weaken and the implant will become loose, requiring revision. Sometimes, the metal of the hip replacement may fracture or the plastics components of it may wear out. In these cases, revision is necessary although in most cases the bone/cement interface usually remains quite strong.

In order to revise any loose or damaged implant, all or most of the plastics cement must be removed before inserting a new prosthesis and re-cementing. Removal of the old cement presents a number of difficulties. It is time-consuming and may cause fracturing of the bone. It involves the careful and tedious use of hand tools such as hammers and cement cutting chisels. High speed burrs have been used, but they frequently perforate the bone and make re-cementing more difficult and not so effective.

It is known from our previous Patent GB 2229660 to provide an ultrasonically powered tool for removal of plastics material. The tool has a planar working surface.

It is an object of the present invention to provide an improved tool for removal of plastics material such as cement from a bore, particularly one in a bone, which overcomes the above disadvantages, by virtue of having a forwardly projecting elongate boring member to facilitate guidance of the tool.

According to the present invention there is provided a tool for use in removing plastics material from a hole comprising a work surface including a substantially annular cutting edge, and adapted to contact said material, piezo electric transducer means operatively connected through a work horn to said work surface to cause it to vibrate ultrasonically and thereby to heat locally said plastics material, cavity means adapted to receive said heated plastics material, means to connect said cavity means to a working zone adjacent said work surface, characterised in that the work surface comprises an elongate boring member and said substantially annular cutting edge is disposed rearwardly thereof, said working zone being connected to said cavity means by means of apertures disposed between said boring member and said annular cutting edge.

Preferably, at least the piezo electric transducer means is sealingly encased in a first enclosure of waterproof plastics material and exterior thereof a second enclosure of stainless steel or the like material.

Advantageously, the waterproof plastics material is an acetal plastics material.

This arrangement allows the tool to be autoclaved or otherwise sterilised for use for another patient.

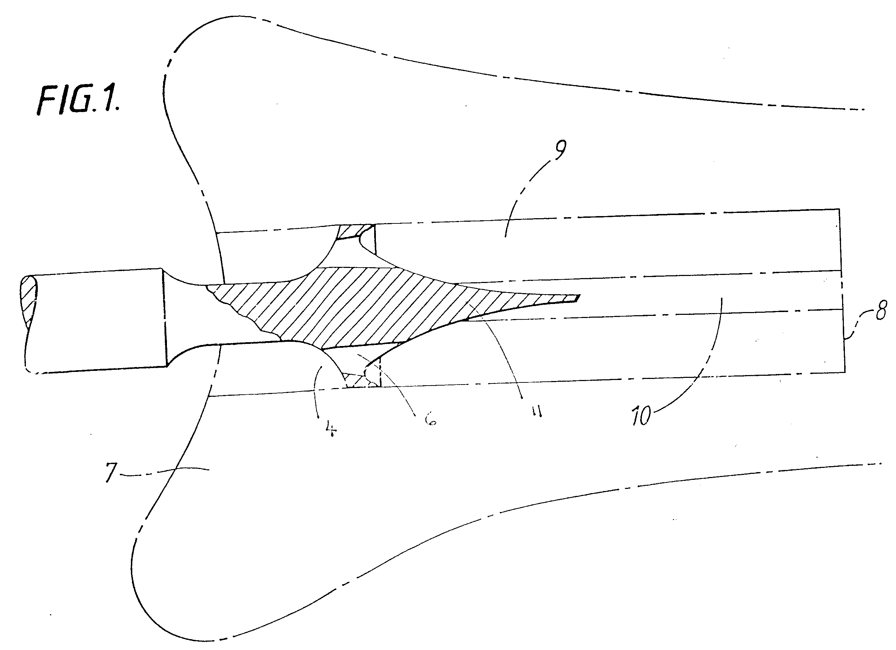

Embodiments of the present invention will now be more particularly described by way of example and with reference to the accompanying drawings, in which:-
FIGURE 1 is a schematic side elevation, shown partially in cross-section, of a working part of a tool embodying the invention;

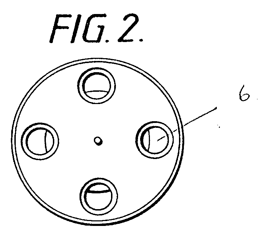

FIGURE 2 is an end elevation of the tool of Figure 1;
FIGURE 3 shows in cross section a drive assembly of the invention;
FIGURE 4 is a cross section of a further embodiment including a handle for use with the invention.

Referring now to the drawings, there is shown in the Figures a tool comprising a piezo electric ceramic transducer 1, connected along a longitudinal axis to a coupling horn 2, which in turn is connected along the longitudinal axis to a work horn 3. At the far end of the work horn 3 is a cavity 4 surrounded by an annular cutting edge 5.

As shown in the Figures, the length of the piezo electric ceramic transducer 1 is half a wavelength, the length of the coupling horn 2 is a full wavelength, while the length of the work horn 3 (which includes the annular cutting edge 5) is an integral number of half wavelengths ensuring that the total probe length can penetrate to the required depth. The term "wavelength" is used to represent the wavelength of the ultrasonic wave generated by the piezo electric ceramic transducer in the material concerned. The preferred material for the work horn and annular cutting edge is titanium or an alloy thereof. At an ultrasonic vibrational frequency in the region of 30-35 kHz, the wavelength of the ultrasonic wave in the titanium alloy is in the region of 70-90mm.

It is well known that many common plastics materials will transmit high frequency vibrations without the significant internal losses which would cause bulk heating of the material. It is also known that when the ultrasound is transmitted through two closely contacted components, the interface can experience a considerable heating effect which under the correct circumstances will produce welding. Such heating can also occur at the interface between a vibrating tool or metal component and the plastics material, the heating being sufficient to melt the plastic. The present invention utilises this effect to drill an annular hole into the plastics material.

The plastics cement material used for hip joint replacements is generally a powder of polymethylmethacrylate beads of diameter less than 100 µm held together in situ by a polymerised methyl methacrylate monomer. This material is prone to creep and is susceptible to localised heating on ultrasonic vibration. The property of creep may be utilised in that, during removal of a core of plastics cement material, the existing cement which remains may be forced into improved engagement with fissures or surface imperfections in the bone by virtue of the ultrasonic vibrations imparted to the cement, and thereby stabilise the interface.

At the work surface, the annular cutting edge can be manipulated by the user of the tool to enable the bore diameter to be widened or, by applying pressure to one side of the tool, to create a hole of oval profile.

The present invention is described with reference to removing a plastics cement from a hip joint replacement during revision of the prosthesis. In this case, the hip bone or femur 7 has a blind hole 8 filled with plastics cement 9 which had originally surrounded the prosthesis, but which has a void 10 where the prosthesis used to be.

In order to operate the tool, the tip 11 is inserted a short distance into the plastics material cement 9 and pushed thereinto for about 5-10mm, as the plastics material softens under the effect of the ultrasonic vibrations. At this point, a core of softened but relatively stiff cement fills the work horn cavity 4.

The sequence is then repeated until the cement is removed from the bore to an appropriate depth. It would be possible to incorporate a small intrascope coaxially within an axial duct in order to facilitate visual inspection of the cutting operation.

Use of the tool embodying the present invention results in a much faster cutting operation and also allows the possibility of leaving intact a thin layer of cement which is characteristically well-bonded to the living bone tissue when revising damaged but not loose implants. If the cement is well-bonded, the strength of the revised implants would be significantly improved. The apparatus also may permit improved bonding between bone and existing cement. Whereas the existing methods of revision of hip joint prosthesis may have required several hours to remove the existing cement, for all of which the patient must be anaesthetised, the present invention allows removal of existing cement, at least sufficient for revision, within a period of less than one hour. The work horn 3 may be curved to suit penetrating of a curved hole in a medulla or similar bone.

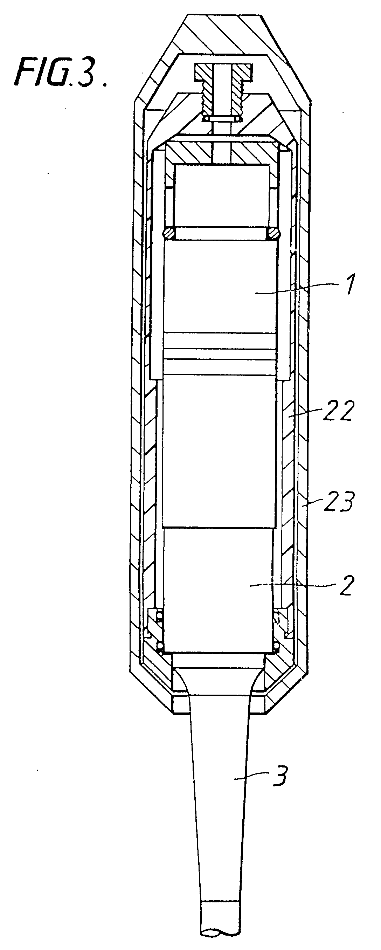

As shown in Figure 3, the piezo electric transducer part of the tool may be encased, first in a layer of acetal plastics material 22 and then in a layer 23 of stainless steel. This arrangement will allow the tool to be autoclaved or otherwise sterilised in order to permit its use on further patients.

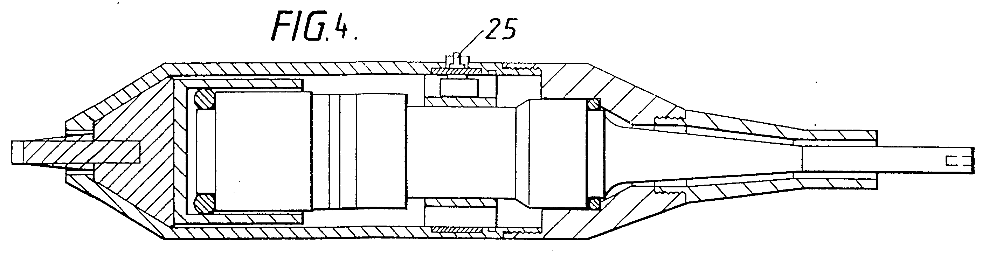

As shown in Figure 4, the handset part of the tool may incorporate a switch 25 and still be autoclavable.

The design philosophy takes account of the conditions prevailing in operating theatres and in particular the strict sterilisation requirements. Several sizes and shapes of oscillatory instrument should ideally be available to the surgeon and a particular case might demand very specialised probe designs. Whereas it is possible to interchange probes on a single handset this procedure is not only inconvenient but results in reduced efficiency of the system if the critical probe/horn interface becomes contaminated with foreign matter. It is desirable therefore to provide a number of independent handsets which can be selected by the surgeon without the need for reconnection or adjustment of switching functions. This dictates the use of a switch incorporated in each handset. Since the handset and cable assembly must be suitable for autoclave sterilisation, the switch assembly requires a special seal design to withstand the temperature and pressure conditions encountered during the sterilisation process,

Figure 4 shows a handset which includes a coaxial switch button operating a sub-miniature micro-switch via a cylindrical moulded seal. The switch is contained in a metal cylindrical sleeve which supports the seal and ensures that it remains water tight even under pressure. This design permits the construction of an oscillatory system offering maximum operating flexibility with inherent reliability. Furthermore there is no need for foot switches which for up to four handsets would involve impractical complications.

## Claims

1. A tool for use in removing plastics material (9) from a hole (8) comprising a work surface including a substantially annular cutting edge (5), and adapted to contact said material, piezo electric transducer means (1) operatively connected through a work horn (2,3) to said work surface to cause it to vibrate ultrasonically and thereby to heat locally said plastics material (9), cavity means (4) adapted to receive said heated plastics material, means to connect said cavity means to a working zone adjacent said work surface, characterised in that the work surface comprises an elongate boring member (11) and said substantially annular cutting edge (5) is disposed rearwardly thereof, said working zone being connected to said cavity means by means of apertures (6) disposed between said boring member and said annular cutting edge (5).

2. A tool as claimed in Claim 1, characterised in that the substantially annular cutting edge (5) is directed forwardly in a similar direction to that in which extends the elongate boring member (11).

3. A tool as claimed in either Claim 1 or Claim 2, characterised in that at least the piezo electric transducer means (1) is sealingly encased in a first enclosure (22) of waterproof plastics material and exterior thereof a second enclosure (23) of stainless steel or the like material.

4. A tool is claimed in Claim 3, characterised in that the waterproof plastics material (22) is an acetal plastics material.

5. A tool as claimed in Claim 3 or Claim 4, characterised in that switch means (25) are provided on a handset of said tool, said switch means (25) being sealed to allow the tool to be autoclaved or otherwise sterilised for use for another patient.

6. A tool as claimed in any one of the preceding claims characterised in that at least one cutting fin is provided to extend radially outwardly of said annular cutting edge.

## Patentansprüche

1. Werkzeug zum Entfernen von plastischem Material bzw. Kunststoff (9) aus einer Bohrung (8), umfassend eine Arbeitsfläche mit einer im wesentlichen ringförmigen Schneidkante (5) für Kontaktierung des Materials, einer piezoelektrischen Wandlereinheit (1), die über ein(en) Arbeitshorn(strahler) (2,3) wirkungsmäßig mit der Arbeitsfläche verbunden ist, um diese in Ultraschallschwingung zu versetzen und damit das plastische Material (9) örtlich zu erwärmen, einem für die Aufnahme des erwärmten plastischen Materials geeigneten Hohlraummittel (4), (und) einem Mittel zum Verbinden des Hohlraummittels mit einer Arbeitszone an bzw. neben der Arbeitsfläche, dadurch gekennzeichnet, daß die Arbeitsfläche ein langgestrecktes Bohrelement (11) umfaßt und die im wesentlichen ringförmige Schneidkante (5) rückwärts davon angeordnet ist, wobei die Arbeitszone mit dem Hohlraummittel über Öffnungen (6), die zwischen dem Bohrelement und der ringförmigen Schneidkante (5) angeordnet sind, verbunden ist.

2. Werkzeug nach Anspruch 1, dadurch gekennzeichnet, daß die im wesentlichen ringförmige Schneidkante (5) in einer ähnlichen Richtung wie die Erstreckungsrichtung des langgestreckten Bohrelements (11) vorwärts gerichtet ist.

3. Werkzeug nach Anspruch 1 oder 2, dadurch gekennzeichnet, daß die mindestens eine piezoelektrische Wandlereinheit (1) abgedichtet in einem ersten Einschluß (22) aus wasserdichtem Kunststoff und außenseitig davon (in) einem zweiten Einschluß (23) aus nichtrostendem Stahl o.dgl. Werkstoff eingekapselt ist.

4. Werkzeug nach Anspruch 3, dadurch gekennzeichnet daß der wasserdichte Kunststoff (22) ein Acetalkunststoff ist.

5. Werkzeug nach Anspruch 3 oder 4, dadurch gekennzeichnet, daß an einem Handapparat (handset) des Werkzeugs Schaltermittel (25) vorgesehen sind, welche Schaltermittel (25) abgedichtet sind, um eine Autoklavenbehandlung oder anderweitige Sterilisierung des Werkzeugs für Verwendung bei einem anderen Patienten zu erlauben.

6. Werkzeug nach einem der vorangehenden Ansprüche, dadurch gekennzeichnet, daß mindestens eine Schneidrippe vorgesehen ist, die von der ringförmigen Schneidkante radial nach außen ragt.

## Revendications

1. Instrument destiné à être utilisé pour enlever de la matière plastique (9) d'un trou (8), qui comprend une surface de travail comportant un bord de coupe (5) sensiblement en anneau et prévu pour entrer en contact avec ladite matière plastique, un moyen transducteur piézo-électrique (1) relié par un pavillon actif (2, 3) à ladite surface de travail pour la faire vibrer à une fréquence ultrasonore et ainsi chauffer localement ladite matière plastique (9), un moyen formant cavité (4) prévu pour recevoir ladite matière plastique chauffée, un moyen pour relier ledit moyen formant cavité à une zone de travail voisine de ladite surface de travail, caractérisé en ce que la surface de travail comprend un élément de perçage allongé (11) et en ce que ledit bord de coupe (5) sensiblement en anneau est disposé vers l'arrière de celle-ci, ladite zone de travail étant reliée audit moyen formant cavité par des ouvertures (6) disposées entre ledit élément de perçage et ledit bord de coupe en anneau (5).

2. Instrument selon la revendication 1, caractérisé en ce que le bord de coupe (5) sensiblement en anneau est orienté vers l'avant dans une direction similaire à celle dans laquelle s'étend l'élément de perçage allongé (11).

3. Instrument selon la revendication 1 ou 2, caractérisé en ce qu'au moins le moyen transducteur piézo-électrique (1) est logé de façon étanche dans un premier boîtier (22) en matière plastique étanche à l'eau à l'extérieur duquel on trouve un second boîtier (23) en acier inoxydable ou en un matériau similaire.

4. Instrument selon la revendication 3, caractérisé en ce que la matière plastique étanche (22) est un matériau plastique acétalique.

5. Instrument selon la revendication 3 ou 4, caractérisé en ce que des moyens de commutation (25) sont disposés sur une poignée dudit outil, lesdits moyens de commutation (25) étant scellés pour pouvoir passer l'instrument à l'autoclave ou le stériliser par un autre moyen afin de l'utiliser avec un autre patient.

6. Instrument selon l'une quelconque des revendications précédentes, caractérisé en ce que au moins une ailette de coupe s'étend radialement vers l'extérieur dudit bord de coupe en anneau.
